Europäisches Patentamt

European Patent Office

Office européen des brevets

(19)

(11) Publication number: **0 132 281**
A2

# (12) EUROPEAN PATENT APPLICATION

(21) Application number: **84303865.4**

(22) Date of filing: **07.06.84**

(51) Int. Cl.⁴: **C 12 P 1/00,** C 07 K 15/00
// C12P21/00, C12N15/00,
G01N33/53 ,(C12P1/00,
C12R1/91)

(30) Priority: **10.06.83 US 503329**
**10.06.83 US 503332**

(43) Date of publication of application: **30.01.85**
**Bulletin 85/5**

(84) Designated Contracting States: **AT BE CH DE FR GB IT
LI LU NL SE**

(71) Applicant: **Martin, Richard F., 510 Oakwood Drive,
Hamilton Ohio 45013 (US)**

(72) Inventor: **Martin, Richard F., 510 Oakwood Drive,
Hamilton Ohio 45013 (US)**

(74) Representative: **Allen, Oliver John Richard et al, Lloyd
Wise, Tregear & Co. Norman House 105-109 Strand,
London, WC2R 0AE (GB)**

(54) Method of isolating membrane antigens from cells.

(57) A method of isolating membrane antigen from pooled
cells comprises homogenizing the pooled cells to provide
a minced mixture of cellular constituents and isolating the
constituent consisting essentially of membrane from the
mixture to generate the membrane antigen.

The membrane constituent may be subjected to gra-
dient purification in order to generate a pooled membrane
antigen which is substantially free of cytoplasmic and nu-
clear contaminants.

ACTORUM AG

1'

## METHOD OF ISOLATING MEMBRANE ANTIGENS FROM CELLS

Monoclonal antibodies are advantageous in that they exhibit specificity and affinity for specific cell populations, such as tumors, within a species. The production of monoclonal antibodies that react with tumors therefore are of significant importance as an analytical tool as well as for diagnosis.

In formulating monoclonal antibodies, it is essential that they bind to and kill the specific selected cell population without affecting normal cells. In addition, the monoclonal antibodies should have a high degree of affinity for such cells. It is believed that the degree of specificity and affinity demonstrated by monoclonal antibodies are dependent upon the purity and availability of the antigen from which the antibodies are produced. Consequently, the purity as well as the availability of the antigen may ultimately limit the usefulness of the monoclonal antibodies produced therefrom. As to the purities of antigen, it is believed that the more purified the antigen, the more likely an antibody can be produced that exhibits a high degree of specificity for synchronous and possibly non-synchronous tumors.

Unfortunately, the antigens generated from the methods heretofore described have failed to produce antigens free from contaminants. For instance, antigens have been derived from membranes of a selected unpooled cell population generated from a single subject according to a centrifugation method described in Thompson et al: The Isolation and Characterization of Tumor-Specific Antigens of Rodent and Human and Tumors. Cancer Research. 36:3518-3525 (Sept. 1976). The antigens formulated therein, however, contain substantial amounts of nucleic and cytoplasmic contaminants affecting and limiting the specificity and affinity of the antibodies produced thereform. Additionally, a pooled colonic crude mixture of cellular constituents has been prepared to generate a tumor associated antigen. Gold, P. and Freedman, S. O.: Specific Carcinoembryonic Antigens of a Human Digestive System. J. Exp. Med. 122:467-481 (1965). Still further, methods to isolate surface membranes which employ glycerol and sucrose gradients have been described. Warren, L.: Membranes of Animal Cells. Method of Isolation of the Surface Membrane. Cell Physiol. 68:269-288 (1966).

It is apparent from the brief overview directed to the importance of monoclonal antibodies to the treatment of tumors that there are continuing needs to generate antigens which are pure and provide

3'

the greatest potential for availability in order to produce therefrom more effective monoclonal antibodies.

In accordance with the invention, a method of isolating membrane antigen from cells comprises obtaining a collection of pooled cells, homogenizing the pooled cells to provide a minced mixture of cellular constituents and isolating constituent consisting essentially of membrane from the mixture to generate the membrane antigen.

Preferably, the membrane constituent may be purified to generate a membrane antigen substantially free of cytoplasmic and nucleic contaminants. To purify the constituent, it is subjected to a gradient such as sucrose or glycerol. In addition, throughout the method, a buffering solution is employed to maintain and protect the cells.

This invention also is directed to the antibodies produced from the antigens generated from the methods of this invention. The antibodies may be produced by any of the known techniques. Membrane antigens are prepared from pooled cells, such as pooled primary human breast carcinoma, to formulate a common membrane antigen.

The invention will now be described, by way of example, with final reference to the accompanying drawings which show electron photomicrographs of various examples.

This invention is directed to a method of isolating membrane antigen from pooled cellular constituents. Preferably, the membrane antigen is substantially free of cytoplasmic and nucleic contaminants. By the term "membrane antigen", it is meant herein that the antigen may be derived from the surface of the cell or the external membrane itself. Additionally, the term "substantially free" in the specification means that the membrane antigen is essentially pure and has a significantly enhanced specificity and activity relatively uncontaminated by cytoplasmic and nucleic components. Further, it is believed that when the membrane antigens are derived from tumor cells, the membrane antigens may be considered "operationally tumor- specific," or tumor associated. For instance, when antibodies are generated from a tumor associated antigen, such as pooled human breast carcinoma, it has been found quite unexpectedly and advantageously that the antibodies are very reactive with a higher percentage of non-synchronous breast cancers and non-responsive to normal breast, heart, kidney, lung and liver cells. Thus, advantageously, essentially purified membrane antigens have been propagated that are capable of generating large amounts of novel monoclonal antibodies which exhibit greater specificity for the cells, i.e., tumor cells, from which the membrane antigens are derived.

This invention further contemplates generating membrane antigens from a collection of pooled cells. By the term "pooled cells" reference is made to a blend of non-synchronous cells, such as primary human breast carcinoma, derived from different sources or subjects. When membrane antigens are derived from pooled cells in accordance with the invention, a broader spectrum of antigens is formed. It has been found quite unexpectedly that the monoclonal antibodies produced from these broader based membrane antigens react with a greater percentage of the non-synchronous cells employed to generate the membrane antigen.

Therefore, the monoclonal antibodies formed from pooled membrane antigens produced in accordance with this invention have not only a great specificity for but also react with a higher percentage of non-synchronous cells from which the membrane antigens are generated. The monoclontal antibodies produced may therefore have general applications directed to cancer treatment, development of laboratory tests, e.g., blood tests, for following patients with proven cancer to determine if additional therapy is indicated, or use in radio imaging to determine the location of the cancer such as metastatic sites. For instance, it is known that antigens shed continuously from cells, such as tumor cells, into the bloodstream. Further, it is believed that a greater percent of the antigens undergoing the shedding process are derived from membranes. It is further believed that the shedding process is accelerated in metastizing tumor cells. Thus, in accordance with the invention operationally tumor-specific membrane antigens can be generated from a pooled collection of such tumor cells to produce corresponding monoclonal antibodies for purposes of diagnostic detection, treatment and/or radio imaging of non-synchronous tumor cells.

Preferably, the invention provides a method of isolating membrane antigen, which is preferably substantially free of cytoplasmic and nucleic constituents, from pooled cells. The method comprises homogenizing a collection of pooled cells to provide a minced or homogenized mixture of cellular constituents, isolating therefrom a constituent consisting essentially of membrane to generate membrane antigen, and, if desired, further purifying the membrane constituent to generate membrane antigen which is substantially free of nucleic and cytoplasmic contaminants.  A presently preferred form of the method comprises dissecting non-synchronous tissue to obtain a collection of selected pooled cells, mechanically homogenizing selected pooled cells to provide a homogenized mixture of cellular constituents, centrifuging the mixture to generate a supernatant substantially free of nuclear constituents and further centrifuging the supernatant to produce a substantially concentrated membrane constituent to generate membrane antigen.  In addition, the membrane constituent may be subjected to gradient purification to generate membrane antigen which is essentially free of cytoplasmic and nucleic contaminants.

The method of the present invention suitably employes a gradient such as sucrose or glycerol, and preferably a sucrose gradient and more preferably a discontinuous sucrose gradient, to purify the cellular constituent consisting essentially of membrane. For instance, purification of the membrane constituent comprises subjecting the membrane constituent to a gradient, such as a discontinuous sucrose gradient, and centrifugation to generate a band containing substantially purified membrane antigen. Thus, the selective use of the purification procedure generates membrane antigens which are essentially free of cytoplasmic and nucleic constituents.

The cells employed to generate the membrane antigen may be derived from a collection of pooled cells such as primary human breast carcinoma. As already discussed, the advantage of pooling cells is to generate a broad spectrum membrane antigen which is operationally-specific for the cancer cells by means of corresponding antibodies produced from the antigen. Specifically, it has been found that the monoclonal antibodies generated from pooled primary human breast carcinoma according to the purification method of this invention have greater specificity for and react with a higher percentage of non-synchronous breast cancers employed to generate the purified pooled membrane antigen.

In a preferred select method, the pooled membrane antigens may be produced as follows. Initially, tissue is dissected from separate sources or subjects to obtain a collection of selected pooled cells. A minced or homogenized mixture of cellular constituents is prepared therefrom. The pooled cells may be mechanically homogenized preferably in a buffer solution, by a homogenizer such as a Brinkman polytron on a setting of about 3 or 4 in about 15 second cycles while employing about 30 second cooling intervals between cycles. The instrument, its setting and time of cycles and cooling intervals may all vary without departing from the teachings of this invention. The buffer employed with this method preferably comprises TRIS(hydroxymethanol)aminomethane hydrochloride, sucrose and mercaptoethanol having about a physiological pH. The range of amounts and pH are variable but it has been found that the cells are best maintained when the buffer contains about 50 mM TRIS(hydroxymethanol)aminomethane hydrochloride, about 250 mM sucrose, about 5mM 2-mercaptoethanol having a pH of about 7.2. Further, it has been found desirable to incorporate therein an antibiotic, such as penicillin and streptomycin, and a preservative like EDTA. It should be understood, however, that any equivalent buffer or mixtures thereof as well as other conventional buffers may be employed in this method as long as the cells are maintained. Generally, it has been found that a buffer ratio of 4 ml buffer to one gram of tumor was most suitable.

Subsequent to homogenizing the cells, the constituent or fraction consisting of essentially membrane may be isolated from the crude or minced mixture by centrifugation. In centrifuging the minced mixture, it is desirable to initially and substantially remove therefrom the nuclear constituent. To accomplish the above, the minced mixture may be centrifuged at about 750 x g up to about 1,500 x g and preferably at about 1,000 x g for a period of about 10 minutes at a temperature of about 2°C up to about 6°C and preferably at about 4°C wherein a first supernatant and pellet are generated. In carrying out the method, the first pellet should be rehomogenized and centrifuged similarly to the procedures already discussed to generate a supernatant and pellet. The supernatant produced up to this point constitutes cellular constituents essentially free of nuclear contaminants. In keeping with the method of this invention, to generate a constituent or fraction consisting essentially of membrane, the first and second supernatants should be combined and centrifuged at about 50,000 x g up to about 200,000 x g, and especially at 75,000 x g for a period of about 60 minutes and at a temperature of about 2°C up to about 6°C and preferably at about 4°C to generate a third supernatant and pellet which constitutes the membrane antigen. Optionally, to maximize the isolation of the constituent consisting essentially of membrane, the third pellet may be washed by centrifugation at about 50,000 x g up to about 200,000 x g, and especially 75,000 x g for about 60 minutes at a temperature of about 2°C up to about 6°C and preferably at about 4°C to generate a fourth supernatant and pellet. In washing the third pellet, it is preferable to employ therewith the buffering solution described herein. However, as stated previously, other suitable buffering solutions may be employed. The resulting fourth pellet constitutes constituent or fraction consisting essentially of membrane. If desired, further steps may be performed and the centrifuging times may be varied without departing from the spirit of this invention.

In furtherance of the method to generate purified membrane antigen substantially free of nucleic and cytoplasmic contaminants, the membrane constituent should be initially resuspended in a solution, such as phosphate buffered saline, and placed on a gradient such as a sucrose or glycerol, and preferably a sucrose gradient and most preferably a discontinuous sucrose gradient. A discontinuous sucrose gradient found to be satisfactory with the method of this invention comprises about 15 ml each of about 40 and about 43% w/w sucrose solutions. Once the membrane constituent has been subjected to the gradient, it should be centrifuged at about 1,200 x g up to about 2,500 x g and preferably at about 1,821 x g for about 120 minutes and at about 2°C up to about 6°C and preferably at about 4°C. The band that is generated should be diluted in a sufficient amount of solution, such as phosphate buffered solution, and centrifuged at about 5,000 x g up to about 15,000 x g and preferably at about 10,000 x g for about 15 minutes at about 2°C up to about 6°C and preferably at about 4°C. Thereafter, the band should be resuspended in a solution-like phosphate buffering solution in an amount sufficient. Once the band has been resuspended, it should be placed on a second gradient preferably a sucrose gradient and more preferably a discontinuous sucrose gradient, for instance, a discontinuous sucrose gradient which comprises about (6 ml each of 50 and 55% w/w sucrose solutions), and centrifuged at about 60,000 x g up to about 130,000 x g and preferably 98,000 x g for about 120 minutes at about 2°C up to about 6°C and preferably at about 4°C. The band should be collected and diluted in a sufficient amount of the preferred phosphate buffered solution and centrifuged at about 5,000 x g up to about 15,000 x g and preferably at about 10,000 x g for about 15 minutes at about 2°C up to about 6°C and preferably at about 4°C. It should be understood, that the bands may be collected by known aspiration techniques, such as a syringe. The resulting pellet constitutes the membrane constituent containing membrane antigen substantially free of cytoplasmic and nucleic contaminants. In keeping with the invention, the purified membrane constituents may be frozen at about -80°C and stored until further use. It should be understood that the time intervals for centrifugation may vary as long as a centrifuged mixture is generated in accord with the spirit of this invention.

In an embodiment, the invention is concerned with the pooled membrane antigen and more preferably the pooled membrane antigen substantially free of cytoplasmic and nucleic contaminants which have been produced in accordance with the method of the invention. Further, the invention is directed to the antibodies generated from these membrane antigens.

As stated, these pooled membrane antigens are basically in a form heretofore believed to be unachieved. Further, when the membrane antigens are purified, such purity may be expressed such that preferably the membrane antigens contain no more than about 10% cytoplasmic and nucleic contaminants. The membrane antigens of this invention are derived from pooled cells which may be derived from the same or different types of tissue or from the same or different sources or subjects. For instance, these membrane antigens contemplate being derived from neoplastic tissues, such as pooled human primary breast carcinoma or other types of human cancer. The produced monoclonal antibodies are advantageously unique in that they are specific and reactive with the cells employed to generate the membrane antigens. In still another feature of this invention, the human cancer cells employed to generate these unique essentially purified membrane antigens membrane antigens may be derived from primary and metastatic cancer cells. Thus, it is additionally possible using the membrane antigens of this invention to generate monoclonal antibodies which can locate the sites of metastatic cancer cells within a human.

Further, a monoclonal antibody is produced from the membrane antigen derived from pooled primary breast carcinoma which is an IgG fraction. It was surprisingly found that this monoclonal antibody is highly reactive and specific for a larger percentage of non-synchronous breast carcinoma cells utilized to generate the antigen.

In another preferred embodiment, pooled membrane antigen preparation of human breast carcinoma is used as a source of membrane from which pooled membrane antigen is obtained, and preferably purified membrane antigen is obtained free from nuclei and cytoplasmic contamination. Whereas it has been known to produce pooled colon tumor cells to generate antigen from a crude mixture of cellular constituents, Gold, P. and Freedman, S.E., supra, it is believed heretofore to be unknown to obtain antigen from membranes of pooled cells. It is further believed heretofore to be unknown to obtain pooled cells of breast carcinoma and antigen therefrom.

In accordance with the invention, the monoclonal antibodies generated from these membrane antigens may be produced from any number of known methods such as disclosed in U. S. Pat. No. 4,172,124. Generally, the method comprises generating membrane antigens of this invention and immunizing an animal, such as a BALB/c mouse, with the membrane antigen. Thereafter, cell hybrids are formed by fusing together antibody producing cells, such as spleen cells or lymph node cells, derived from the animal with myeloma cells. The hybridomas are cloned to generate clones which produce antibodies that demonstrate a specificity for the tumor cells. It should be understood, however, that the monoclonal antibodies produced from the "unpurified" membrane antigen in accordance with this invention may exhibit, in addition to their high specificity and affinity for membrane, a limited specificity for cytoplasm.

A number of Examples will now be outlined, which illustrate the preparation of a pooled membrane antigen and corresponding monoclonal antibody.

## EXAMPLE I

Normal and neoplastic tissue antigen preparations were prepared. At the time of mastectomy, grossly and microscropically confirmed breast carcinoma was dissected free of normal tissue. At the same time, normal breast tissue at least 2 cm from the primary tumor, uninvolved non-cancerous breast tissue, which contained no microscopic carcinoma was dissected entirely free of fat tissue. Approximately equal proportions of breast carcinoma tissue from 10 individuals were pooled and minced in homogenizing buffer (50 Mm TRIS(hydroxymethyl)aminomethane hydrochloride, pH 7.2, with 250 Mm sucrose, 1 Mm EDTA, 5 Mm 2-mercaptoethanol, 100 U/ml penicillin, and 100 µg/ml streptomycin at a ratio of 4 ml buffer/gram of tumor. The breast carcinoma tissue pool was homogenized using a Brinkman polytron on setting 3 or 4, 15-second cycles with 30-cooling intervals between cycles. This crude tumor tissue homogeneate was centrifuged at 1,000 x g for 10 minutes at 4°C. The supernatant was retained and the pellet rehomogenized and centrifuged as before. The two supernatants were pooled and centrifuged at 75,000 x g for 1 hour at 4°C in the homogenizing buffer.

Normal tissue from the same ten individuals used in the breast carcinoma tissue preparation was employed to make a pooled crude normal breast antigen preparation using the same buffer as above. Approximately equal portions of the non-cancerous breast tissue was pooled and minced in the homogenizing buffer. The tissue was homogenized using a Brinkman polytron on setting 3 or 4 for 15-second intervals with 30-second cooling between cycles and subsequently lypholized and stored at room temperature until use. This tissue was designated as normal pooled breast tissue membrane antigen preparation. In addition, other tissue preparations including fibroadenoma (pool of 5 patients), spleen (pool of 5 patients) were prepared in the same fashion.

A monoclonal antibody was then prepared from the breast carcinoma membrane antigen and tested against unrelated tumors and normal tissue as follows.

Immunization and Bleeding of Mice

BALB/c mice were injected intraperitoneally (IP) with 400 µg (total protein) of the tumor membrane antigen preparation in equal volumes of PBS and complete Fruend's adjuvant. Six weeks later, the mice were injected IP with 100 µg of the same membrane antigen in soluble form. Three days following the injection of the soluble membrane antigen, the spleen was removed for fusion. Antibody titration on the day of fusion was 1:400.

Cell Fusion

Cell fusion was accomplished by a modification of the procedure of Oi and Herzenburg with a spleen to myeloma cell fusion ratio of 1:1 using X63/AG 653 myeloma cell line. Equal portions of myeloma cells and spleen cells were combined in a 50 ml centrifuge tube and sedimented by centrifugation at 400 x g for 10 min at 22°C. All procedures and reagents were maintained at 37°C for the remainder of the fusion procedure. One ml of 50% PEG solution containing 2 g of 4,000 MW PEG, 2 ml serum-free DMEM, and 0.2 ml dimethylsulfoxide was added to the pellet over a 1 minute period with gentle mixing. The reaction was slowly stopped during the next 5 minutes by the gradual addition of 8 ml serum-free DMEM. The cells were sedimented by centrifugation at 400 x g for 10 minutes at 22°C and the supernatant was discarded. The pellet was dispersed into fine clumps by vigorous addition of 10 ml DMEM containing 20% fetal calf serum, 10 mM hypoxanthine, 0.04 mM aminopterin, and 1.6 mM thymidine (DMEM-HAT). An additional 20 ml DMEM-HAT was added and 0.1 ml of the suspension was distributed into individual wells of 96-well tissue culture plates (Costar, Cambridge, MA) which contained at least $3 \times 10^3$ mouse peritoneal macrophages in 0.05 ml DMEM-HAT per well. The plates were incubated in 8-10% $CO_2$ atmosphere at 37°C.

Selection of Hybrids

At 14 days, 10 hybridoma supernatants were considered positive against the membrane preparation as tested by ELISA described below and were transferred into 1 ml wells. These were rescreened at 21 days and two remained positive against the membrane

20'

preparation. Subsequently, one hybrid remained positive and viable after limiting dilutions and ascites tumor production.

Feeder Cells

Feeder macrophage cells were harvested according to the technique of Fazekasas as follows: mouse abdominal skin was scrubbed and prepared with both alcohol and then Betadine. The mice were injected with 11% sucrose solution containing 1% penicillin and streptomycin and after gentle massage a minimum of 200,000 macrophages per ml were obtained. A minimum of 3,000 macrophages were distributed in 96 well plates as feeder cells as well as 30,000 prior to transfer of hybrids into 1 ml wells, 100 to 200,000 macrophage feeder cells were used as feeder cells prior to transfer into 25 cm or 75 cm flasks.

Propagation of Hybrids

The hybrids were cloned by limiting dilutions and an average of approximately 5, 2, 1 and less than 1 hybrid per well were distributed into 96 well culture 0.2 ml wells following distribution of a minimum of 3,000 feeder macrophages. Positive hybrids were again cloned by limiting dilution, retested and expanded.

BALB/c mice were primed with Pristane

21'

(Aldridge) and injected with the hybrid cells grown in culture.  Ascitic fluid was spun at 400 X g for 10 minutes and retested.  Both the tumor and the ascitic fluid pellet were resuspended in 90% fetal calf serum with 10% DMSO and frozen and stored at either -70° or -80°C in liquid nitrogen.

Enzyme Linked Immunoabsorption Assay (ELISA)

The total protein content of all screening antigens was measured by the coomassie blue dye binding method of Bradford (Environmental Chemical Specialties, Inc., Anaheim, CA).  The protein content of all antigens used in ELISA was adjusted to 1 µg/ml in carbonate buffer (pH 9.6, 15 mM $Na_2CO_3$, 35 mM $NaHCO_3$, 3 mM $NaN_3$).  The antigens in the wells were then dried overnight in a nonhumidified incubator at 37°C.  The wells were then washed 3 times with PBS containing 0.5 ml Tween 20:1 (PBST).  Two hundred 1 of PBST containing 10 mM EDTA (PBST-EDTA) and 0.5% globulin free bovine serum albumin (BSA) were added to each well and the plates were incubated at 37°C for 1 hour.  The contents of each well were removed by aspiration and the wells were washed 5 times with PBST-EDTA.  Fifty µl of hybridoma supernatant or 50 µl of mouse ascites fluid or serum (diluted 1:20 in PBST) to be tested was added to appropriate wells.

22'

After incubation at 37°C for 1 hour in an 8-10% $CO_2$ atmosphere, the contents of each well were removed by aspiration. The wells were washed once with PBST-EDTA containing 0.1% BSA and 4 times with PBST, pH 7.4, containing 1% BSA. Goat anti-mouse IgG horseradish peroxidase conjugate (Tago, Burlingame, CA) diluted 1:2000 in PBST (pH 7.4) was prepared immediately prior to use and 50 μl of the conjugates was added to the test wells. After 1 hour incubation at 37°C, the fluid was removed by aspiration and each well was washed 3 times with PBST, pH 7.4, containing 1% BSA and twice with distilled water. One hundred μl of 0-phenylenediamine was added to each well. After 1 hour incubtion at 22°C in the dark, the OPD reaction was stopped by the addition of 700 μl of 1.3 N $H_2SO_4$ and the samples were read on a Stasar Gilford spectrophotometer at a wavelength of 492 nm. The negative controls consisted of an antigen control (antigen + conjugate + OPD), a supernatant or ascites fluid control (hybridoma supernatant or ascites fluid diluted 1:20 + conjugate + OPD), an OPD control (OPD alone) and a conjugate control (conjugate + OPD). The positive control consisted of a 1:20 dilution of mouse serum known to generate a positive reaction with one of the screening antigens.

23'

## Immunoelectrophoresis

The Corning-ACI agarose Film Cassette System with Corning ACI Barbital buffer was used to test ascitic fluid as follows: 1.0 µl of undiluted or 1:20 diluted ascitic fluid was applied and was subjected to electrophoresis for 30 minutes. Appropriate anti-sera [affinity purified goat anti-mouse IgG light chain specific, anti 2A, anti 2B, anti-3, anti $G_1$ anti IGM (Tago) and kappa and lambda] were applied in the anti-sera troughs in two, 40 µl applications. After 48 to 72 hours at room temperature, reactions were read and photographed.

## Immunoperoxidase

Sections of snap frozen tissue were adhered to glass slides in the cryostat at -20°C without initial acetone fixation. Subsequently, they were stored at -70°C and fixed in cold acetone just prior to use. The slides were placed in a 37°C oven for ½ hour and at room temperature up to 30 minutes. Thereafter, they were incubated in PBS at 3 minutes each. Sections were incubated with a 1:20 to 1:2000 dilution of ascitic fluid or affinity purified ascitic fluid for 60 minutes. This was followed with three changes of PBS at 3 minutes each. Sections were incubated with goat, anti-mouse IgG peroxidase conjugated (Tago) 1/30 in PBS for 15 minutes and following this rinsed with three changes of PBS, 3 minutes each. They were incubated in AEC solution for 15 minutes and rinsed in distilled water for 3 minutes. They were stained with progressive hematoxylin for 2 minutes and mounted with glycerine jelly. Control sections reacted with PBS and were run parallel to each tissue. Additional controls included, non-immune mouse serum (diluted appropriately), conjugate control (no conjugate), AEC control with each experiment.

Antibody neutralization studies of ascitic fluid, undiluted, 1/5, 1/10, 1/20 and 1/40 absorbed with 4+ positive lypholized breast cancer and normal lypholized breast demonstrated an abolition of antibody activity, but no change in reactivity after reaction with normal breast tissue as tested in the immunoperoxidase reaction above.

Results of Activity and Specific Tests

The monoclonal antibody, identified as 2C10, was an IgG, 2A kappa monoclonal antibody produced from the membrane antigen generated from pooled human primary breast carcinoma which in ELISA testing reacted to 10 out of 11 non-related, non-synchronous primary tumors (99%). Likewise, enzyme linked immunoassay testing against normal pooled non-cancerous

25'

breast tissue from the same patients whose tumors served as membrane preparations were negative with 2C10. It did not react to either normal spleen, fibroadenoma or normal serum.

Immunoperoxidase studies using frozen sections of 15 non-synchronous unrelated primary tumors all reacted to 2C10 (100%). There was strong to intense membrane or apical staining in 8 out of the 15 and minimal to moderate staining in the remainder. Negative staining reactions were observed in the immunoperoxidase to fibroadenoma, non-cancerous normal breast tissue from mastectomy specimens and normal breast tissue removed at the time of breast biopsy. In addition to non-reactions to these normal tissues, this monoclonal antibody reacts to primary and metastatic carcinoma of the same patient and has reacted positively with breast cancer cells in pleural effusion. 2C10 does not react to other normal human tissues, including lung, liver, or heart.

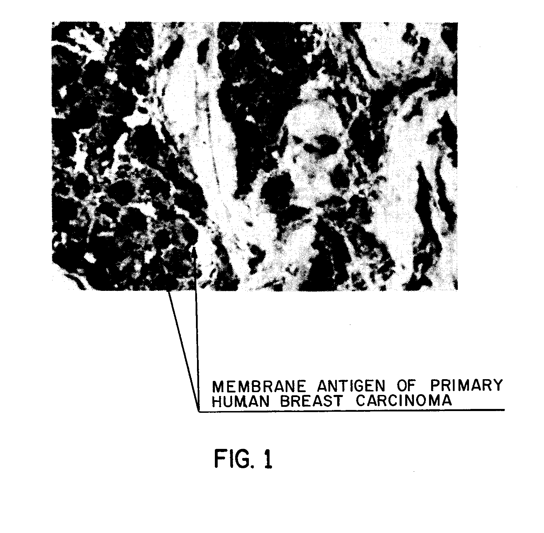

The significance of these results may be exemplified and highlighted with reference to the electron photomicrograph of the drawing in figure 1.

Figure 1 is a photomicrograph enlarged at about 400 times of a frozen section of human breast carcinoma using the immunoperoxidase technique described above showing by arrows membrane and cytoplasm localization of the monoclonal antibody 2C10 in the human breast cancer. This photograph demonstrates the activity and specificity for the cell, particularly the cell membrane and possibly the cytoplasm by the distribution of antibody activity on the membrane and cytoplasm of the cancer cell. This is highlighted in the photograph by the small spotty black areas shown by arrows designated as A.

EXAMPLE II

Normal and neoplastic tissue antigen preparations were prepared. At the time of mastectomy, grossly and microscropically confirmed breast carcinoma was dissected free of normal tissue. At the same time, normal breast tissue at least 2 cm from the primary tumor, uninvolved non-cancerous breast tissue, which contained no microscopic carcinoma was dissected entirely free of fat tissue. Approximately equal proportions of breast carcinoma tissue from 10 individuals were pooled and minced in homogenizing buffer (50 Mm TRIS (hydroxymethyl) aminomethane hydrochloride, pH 7.2, with 250 Mm sucrose, 1 Mm EDTA, 5 Mm 2-mercaptoethanol, 100 U/ml penicillin, and 100 µg/ml streptomycin) at a ratio of 4 ml buffer/gram of tumor. The breast carcinoma tissue pool was homogenized using a Brinkman polytron on setting 3 or 4, 15-second cycles with 30-cooling intervals between cycles. This tumor tissue was centrifuged at 1,000 x g for 10 minutes at 4°C. The supernatant was retained and the pellet rehomogenized and centrifuged as before. The two supernatants were pooled and centrifuged at 75,000 x g for 1 hour at 4°C in the homogenizing buffer. The pellet was washed once by centrifugation at 75,000 x g for 1 hour at 4°C in homogenizing buffer. At this point, a pooled unpurified membrane preparation of human breast carcinoma had been prepared.

The membrane preparation was resuspended in phosphate buffered saline and placed on a discontinuous sucrose gradient (15 ml each of 40 and 43% w/w sucrose solutions) and centrifuged at 1,821 x g at 4°C for 2 hours. The band was collected by syringe, diluted in 5 ml of phosphate buffered saline, centrifuged at 10,000 x g for 15 minutes at 4°C and resuspended in 5 ml of phosphate buffered saline. The preparation was placed on a second discontinuous sucrose gradient (16 ml each of 50 and 55% w/w sucrose solutions) and centrifuged at 98,000 x g for 2 hours at 4°C. The band was collected by aspiration, diluted in 2 ml of phosphate buffered saline and centrifuged at 10,000 x g for 15 minutes. An aliquot was removed for electron microscopy and the remainder frozen at -80°C.

Normal tissue from the same ten individuals used in the breast carcinoma tissue preparation was homogenized using a Brinkman polytron on setting 3 or 4 for 15-second cycles with 30-second cooling intervals between and this tissue was designated as normal breast tissue antigen preparation. In addition, other tissue preparations including fibroadenoma (pool of 5 patients), spleen (pool of 5 patients) were prepared in the same fashion.

A monoclonal antibody was then prepared from the breast carcinoma membrane antigen and tested against unrelated tumors and normal tissue as follows.

Immunization and Bleeding of Mice

BALB/c mice were injected intraperitoneally (IP) with 300 g (total protein) of the membrane antigen preparation in equal volumes of PBS and complete Freund's adjuvant. Six weeks later, the mice were injected intraperitoneally with 100 µg of the same membrane antigen in soluble form. Three days following the injection of the soluable membrane antigen, the spleen was removed for fusion. On the day of fusion, antibody titration studies revealed a titer of 1:800.

## Cell Fusion

Cell fusion was accomplished by a modification of the procedure of Fazekasas with a spleen to myeloma cell fusion ratio of 1:1 using X63/AG 653 myeloma cell line. Equal portion of myeloma cells and spleen cells were combined in a 50 ml centrifuge tube and sedimented by centrifugation at 200 x g for 10 min at 22°C. All procedures and reagents were maintained at 22°C except where indicated. One ml of 50% PEG, prewarmed to 45°C, was added to the cell pellet over a 1 min period, then the tube was immersed for 90 seconds in a 37°C water bath. The reaction was slowly stopped during the next 3 minutes by the gradual addition of 20 ml serum-free DMEM. An additional 25 ml serum-free DMEM was added to the tube, and the mixture allowed to stand for 5 minutes. The cells were sedimented by centrifugation at 200 x g for 10 minutes at 22°C and the supernatant was discarded. The pellet was resuspended in DMEM-HAT to a final concentration of 4-6 x $10^6$ cells/ml and 0.05 ml of the suspension was distributed into individual wells of 96-well tissue culture plates which contained at least 7 x $10^3$ mouse peritoneal macrophages in 0.1 ml DMEM-HAT per well. The plates were incubated in an 8-10% $CO_2$ atmosphere at 37°C. After extensive screening by ELISA testing described below, one hybrid was expanded into 25 cm and 75 cm flasks for ascites production after double limiting dilutions.

Propagation of Hybrids

The hybrids were cloned by limiting dilutions and an average of approximately 5, 2, 1 and less than 1 hybrid per well were distributed into 96 well culture, 2 ml wells following distribution of a minimum of 3,000 feeder macrophages. Positive hybrids were again cloned by limiting dilutions, retested and expanded.

BALB/c mice were primed with Pristane (Aldridge) and injected with the hybrid cells grown in culture. Ascitic fluid was spun at 400 x g for 10 minutes and retested. Both the tumor and the ascitic fluid pellet were resuspended in 90% fetal calf serum with 10% DMSO and frozen and stored at either -70° or -80°C in liquid nitrogen.

Cloning of Hybridomas in Soft Agarose

Feeder macrophages were obtained and placed in 16 mm tissue culture dishes and fibroblasts were added until they were approximately 1/4 to 1/2 confluent. Subsequently, 5 ml of agarose and cloning media containing 20% cloning fetal calf serum 10% NCTC 109, 1% pen-strep was used for the feeder underlayer.

Cell layer was applied 24 hours later and contained 0.8 ml of an agarose media mixture with 0.2 ml of growing hybrids in 37°C media. The cells were added drop by drop leveled at 4° for 10 minutes and placed in the $CO_2$ incubator. Four days later, the cells were fed with the original dilution of agarose and media, 0.8 ml agarose media and 2 ml goat anti-mouse IgG (Azide free, Tago). Clones showing percipitant lines at approximately 4 to 6 days were subsequently removed from the soft agar using a finely bent Pasteur pipette and re-expanded and retested.

Enzyme Linked Immunosorbent Assay (ELISA)

The specificity of monoclonal IgG antibodies produced by hybridomas in tissue culture or mouse ascites fluid was defined by ELISA. The total protein content of all screening antigens was measured by the coomassie blue dye binding method of Bradford. The protein content of all antigens used in the ELISA was adjusted to 1 µg/ml in carbonate buffer (pH 9.6, 15 mM $Na_2CO_3$, 35 mM $NaHCO_3$, 3 mM $NaN_3$). Dynatech 96 well polystrene plates were moistened with distilled water, drained well, and 50 µg of test antigen was added to appropriate wells. Serum used as test antigen was diluted 1:3 in carbonate buffer, and 50 µl diluted serum was added to appropriate wells of a 96-well plate. The antigens in the wells were then dried overnight in a non-humidified incubator at 37°C. The wells were then washed 3 times with PBS containing 0.5 ml Tween 20:1 (PBST). Two hundred μl of PBST containing 10 mM EDTA (PBST-EDTA) and 0.5% globulin-free bovine serum albumin (BSA) were added to each well and plates were incubated at 37°C for 1 hour. The contents of each well were removed by aspiration and the wells were washed 5 times with PBST-EDTA. 50 μl of hybridoma supernatant or 50 μl of mouse ascities fluid or serum (diluted 1:20 in PBST) to be tested was added to appropriate wells. After incubation at 37°C for 1 hour in an 8-10% $CO_2$ atmosphere, the contents of each well were removed by aspiration. The wells were washed once with PBST-EDTA containing 0.1% BSA and 4 times with PBST, pH 7.4, containing 1% BSA. Goat anti-mouse IgG horseradish peroxidase conjugate (Tago, Burlingame CA) diluted 1:2000 in PBST (pH 7.4) was prepared immediately prior to use, and 50 μl of the conjugate was added to the test wells. After 1 hour incubation at 37°C, the fluid was removed by aspiration and each well was washed 3 times with PBST, pH 7.4 containing 1% BSA and twice with distilled water. 100 μl of 0-phenylenediamine was added to each well. The OPD working solution was prepared 20 minutes prior to use by adding 0.4 mg OPD per ml $H_2O_2$/citrate buffer prepared using a 1:100 ratio of 3% $H_2O_2$ to citrate buffer (24 mM citric acid, 51 mM $Na_2HPO_4$, and 100 mg/l merthiolate, pH 5.0). After 1 hour incubation at 22°C in the dark, the reaction was stopped by the addition of 700 μl of 1.3 N $H_2SO_4$, and the samples were read on a Stasar Gilford spectrophotometer at a wavelength of 492 nm. The negative controls consisted of an antigen control (antigen + conjugate + OPD), a supernatant or ascites fluid control [hybridoma supernatant or ascites fluid diluted 1:20 + conjugate + OPD, and OPD control (OPD alone), and a conjugate control, conjugate + OPD].

Immunoperoxidase

Four micron cryostat sections were mounted directly onto glass slides and transferred on dry ice and stored at -70°C. The slides were placed in a 37°C oven for 1/2 hour at room temperature for up to 30 minutes. Immediately prior to processing, sections were fixed in cold acetone for 5 minutes and allowed to fully dry at room temperature. The slides were incubated in PBS for 3 minutes and subsequently a 1:20 to 1:2000 dilution of ascitic fluid or affinity purified ascitic fluid for 60 minutes following three changes of PBS, x 3 minutes each, the sections were

0132281

34'

incubated in goat anti-mouse IgG affinity, purified peroxidase conjugated (Tago) 1:30 in PBS for 60 minutes.  Following three changes of PBS each of three minutes, the sections were incubated in AEC solution for 15 minutes.  Following rinsing in distilled water, they were counterstained with progressive hematoxylin and mounted in glycerin jelly.  Control sections reacted with PBS and were run parallel to each tissue. Additional controls included, non-immune mouse serum (diluted appropriately), conjugate control (no conjugate), AEC control with each experiment.

Antibody neutralization studies of ascitic fluid, undiluted, 1/5, 1/10, 1/20, and 1/40 absorbed with 4+ positive lypholized breast cancer and normal lypholized breast demonstrated an abolition of antibody activity, but no change in reactivity after resection with normal breast tissue as tested in the immunoperoxidase reaction above.

Immunoelectrophoresis

The Corning-ACI agarose Film Cassette System with Corning ACI Barbital buffer was used to test ascitic fluid as follows:  1.0 µl of undiluted or 1:20 diluted ascitic fluid was applied and was subjected to electrophoresis for 30 minutes.  Appropriate anti-sera [affinity purified goat anti-mouse IgG light chain specific, anti 2A, anti 2B, anti-3, anti $G_1$, anti IGM (Tago) and kappa and lambda] were applied in the anti-sera troughs in two, 40 µl applications. After 48 to 72 hours at room temperature, reactions were read and photographed.

Results of Activity and Specificity Tests

The monoclonal antibody, identified as 7G5, was an IgG 2A, kappa monoclonal antibody produced from the membrane antigen generated from pooled human primary breast carcinoma which in ELISA tested against 8-out of 10 (80%) non-related, non-synchronous primary tumors. In addition, it reacted to metastatic tissue present in lymph node. Likewise, enzyme linked immunoassay testing against normal pooled non-cancerous breast tissue from the same patients were negative with 7G5. It did not react to either normal spleen or fibroadenoma.

Immunoperoxidase studies using frozen sections of 15 non-synchronous unrelated primary tumors all reacted to 7G5 (100%). There was strong to intense membrane or apical staining in 9 of the 15 and moderate staining in 3 with only minimal staining reaction in 3 of the 15 patients. Weak to negative staining reactions were observed in the immunoperoxidase to fibroadenoma, non-cancerous normal breast tissue from mastectomy specimen and normal breast tissue removed at the time of breast biopsy. In addition to non-reactions to these normal tissues, this monoclonal antibody reacted to primary and metastatic carcinoma of the same patient and had reacted positively with breast cancer cells in pleural effusions. 7G5 did not react to other normal human tissues tested in the immunoperoxidase including lung, liver, heart and kidney.

The significance of these results may be exemplified and highlighted with reference to the following figures of electron photomicrographs of the drawing.

The electron photomicrographs of the drawing were prepared by taking a specimen of the preparation under consideration and first fixing it in a fluid paraformaldehyde (45 paraformaldehyde in phosphate buffer). Then the resulting preparation was osmicated with 2% solution of osmium chromate. Thereafter, it was dehydrated through a series of acetone solutions and then embedded in SPURR (trade name for an embedding plastic). After embedding the preparation in the plastic, the plastic was polymerized overnight to form blocks. The blocks were then sectioned using a Diamond Microton into ultra thin sections and stained with uranyl acetate and lead citrate. The sections were then viewed in a Zeiss 109 electron microscope under the magnifications indicated and photographed on Ilford film ASA 50.

Fig. 2 is an electron photomicrograph of the membrane preparation prepared according to the method of Thompson et al, supra, at a magnification of 20,000 times.

Fig. 3 is an electron photomicrograph of the membrane antigen of primary human breast carcinoma as purified in accordance with the method of this invention.

Fig. 4 is an electron photomicrograph enlarged about 500,000 times of a purified membrane antigen prepared in accordance with the method of this invention, similar to that shown in Fig. 3, to verify that the area indicated in Fig. 3 by an arrow is a purified membrane antigen. Fig. 4A is an increased magnification of the trilaminar unit cellular membrane of approximately 60° to 70° Angstroms in width shown in Fig. 3.

Fig. 5 is a photomicrograph enlarged at about 400 times of a frozen section of human breast carcinoma using the immunoperoxidase technique described above showing by arrow B the membrane localization of the monoclonal antibody 7G5 in the human breast cancer. This photograph demonstrates the antibody activity and high degree of specificity for the cell, particularly the cell membrane by the linear deposition of antibody activity on the membrane of the cancer cell. This is highlighted in the photograph by the rather continuously black area shown by arrow B.

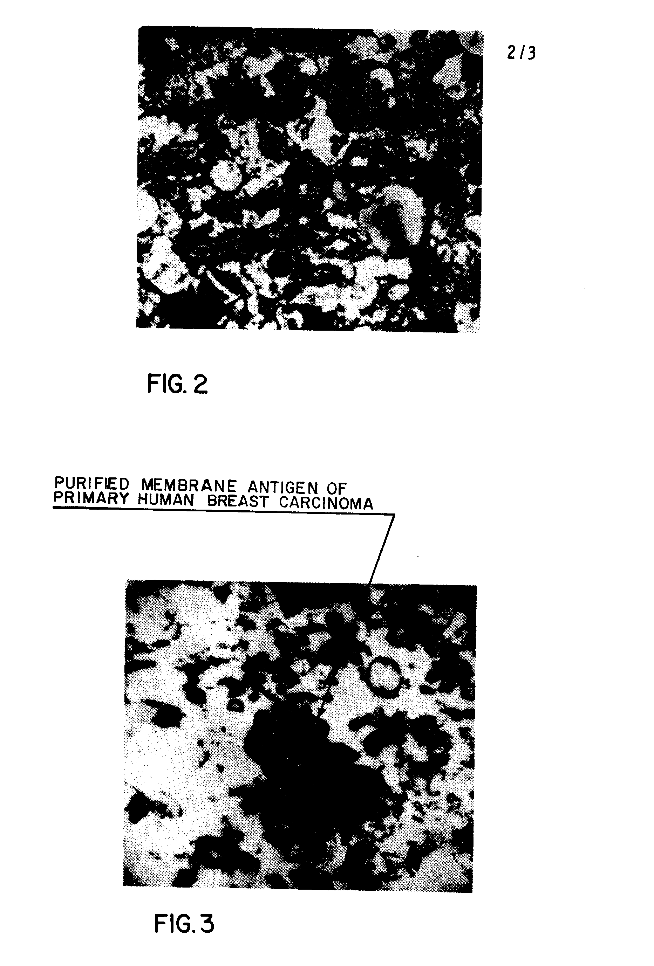

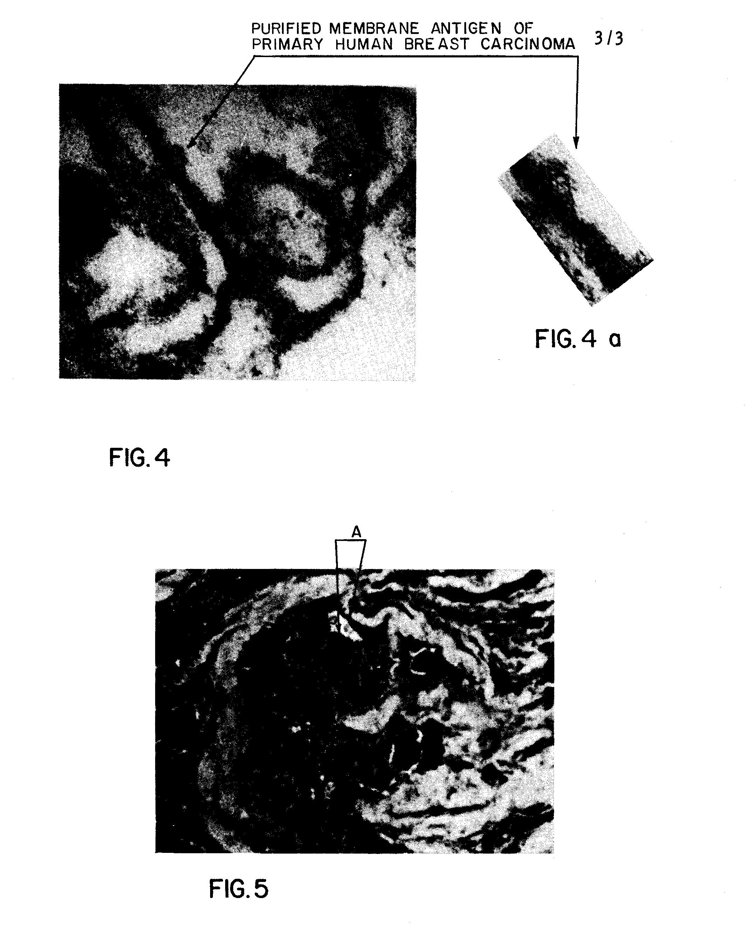

With reference to Figs. 2-5, the invention is distinguished from known methods such as that of Thompson et al by providing essentially pure membrane antigen. By comparing Fig. 2 with Fig. 3, one may ascertain the high degree of purity or substantial freedom from cytoplasmic and nucleic contamination achieved of the membrane antigen of this invention. Figs. 4 and 5 demonstrate the specificity of the antibody produced from the membrane antigen for the membrane of the cancer cell in a remarkable manner.

The present invention may, of course, be carried out in other specific ways than those herein set forth without departing from the spirit and essential characteristics of the invention. The present embodiments are, therefore, to be considered in all respects as illustrative and not restrictive and all changes coming within the meaning and equivalency range of the appended claims are intended to be embraced herein.

39'

CLAIMS:

1.　　　　A method of isolating membrane antigen from cells comprising obtaining a collection of pooled cells, homogenizing the pooled cells to provide a minced mixture of cellular constituents and isolating the constituent consisting essentially of membrane from the mixture to generate the membrane antigen.

2.　　　　A method as claimed in Claim 1 wherein the cells are homogenized in a buffer.

3.　　　　A method as claimed in Claim 2 wherein the buffer comprises TRIS, sucrose and mercaptoethanol.

4.　　　　A method as claimed in either Claim 2 or 3 wherein the buffer includes an antibiotic.

5.　　　　A method as claimed in any preceding Claim wherein the constituent consisting essentially of membrane is isolated from the mixture by centrifugation.

6.　　　　A method as claimed in any preceding Claim which further comprises producing antibodies that correspond to the membrane antigens.

7.      A method as claimed in any preceding claim wherein the isolation of the constituent consisting essentially of membrane to generate the membrane antigen comprises centrifuging the minced mixture between 750 and 1,500 x g at a temperature between 2 and $6^{o}$C to generate a first supernatant and a pellet, subjecting the pellet to homogenization and centrifuging between 750 and 1500 x g at a temperature between 2 and $6^{o}$C to generate a second supernatant, combining the first and second supernatants, and centrifugating the combined supernatants between 50,000 and 120,000 x g at a temperature between 2 and $6^{o}$C to generate a third supernatant and pellet, wherein the third pellet constitutes constituents consisting essentially of membrane to produce the membrane antigen.

8.      A method as claimed in any preceding Claim wherein the pooled cells are derived from primary human breast carcinoma.

9.      A method as claimed in any preceding Claim comprising subjecting the membrane constituent to gradient purification to generate a pooled membrane antigen which is substantially free of cytoplasmic and nuclear contaminants.

10.     A method as claimed in Claim 9 wherein the gradient is either sucrose or glycerol.

MEMBRANE ANTIGEN OF PRIMARY
HUMAN BREAST CARCINOMA

FIG. 1

0132281

2/3

FIG. 2

PURIFIED MEMBRANE ANTIGEN OF
PRIMARY HUMAN BREAST CARCINOMA

FIG. 3

PURIFIED MEMBRANE ANTIGEN OF
PRIMARY HUMAN BREAST CARCINOMA

FIG. 4 a

FIG. 4

A

FIG. 5